# FASCICULE DE BREVET EUROPEEN

(11) **EP 1 488 754 B1**
(45) Date de publication et mention de la délivrance du brevet: **29.03.2017**
(21) Numéro de dépôt: 04356097.8
(22) Date de dépôt: 14.06.2004
(51) Int. Cl.: A61B 17/80, A61B 17/70, F16B 39/10

(54) **Dispositif destiné à être accouplé à au moins un support, et notamment implant chirurgical destiné à être accouplé à un os**
Vorrichtung zum Verbinden mit wenigstens einem Halter, insbesondere chirurgisches Implantat zum Verbinden mit einem Knochen
Device to be coupled to at least one holder, in particular surgical implant to be coupled to a bone

(30) Priorité: 16.06.2003 FR 0307237
(43) Date de publication de la demande: 22.12.2004
(73) Titulaire: Newdeal, 69006 Lyon (FR)
(72) Inventeur: Forcault, Eric Stéphane, 69004 Lyon (FR); Knevels, Theo Jan Maria, 1850 Grimbergen (BE); Giet, Jean-Christophe Alain, 69006 Lyon (FR); Gauneau, Bertrand Xavier François, 69007 Lyon (FR)
(74) Mandataire: Martin, Didier Roland Valéry

(56) Documents cités:
- EP-A- 0 345 133
- WO-A-99/59492
- FR-A- 2 790 198
- US-B1- 6 235 033

## Description

La présente invention se rapporte au domaine technique des dispositifs destinés à être solidarisés à un support, en vue par exemple de consolider ou de réparer ledit support, ou encore pour doter ce support d'un élément complémentaire fonctionnel et/ou décoratif constitué par le dispositif.

La présente invention concerne un dispositif destiné à être accouplé à au moins un support, ledit dispositif comprenant :
- un organe de fixation comportant une tête et une tige destinée à être ancrée dans le support,
- une pièce d'assemblage comprenant au moins un trou débouchant,
- une bague logée de manière orientable au sein dudit trou débouchant, ladite bague étant munie d'un passage conformé pour d'une part permettre le passage de la tige et d'autre part pour réaliser un moyen d'arrêt pour la tête.

L'invention est plus particulièrement dirigée vers un dispositif chirurgical, du genre implant, destiné à être assemblé à un os ou à des fragments osseux, mais elle peut également s'appliquer à tout autre domaine, tel que le bâtiment ou le bricolage.

Dans ce qui suit, à titre purement illustratif et non limitatif, on fera référence généralement au domaine médical.

On connaît par le document EP-345 133 un implant chirurgical destiné à être assemblé à un os à l'aide d'une vis. L'implant chirurgical décrit dans ce document comprend un passage à travers lequel passe la vis, et un lamage fileté dont le diamètre est supérieur à celui de la tête de la vis, de façon à définir un fond constituant une surface d'appui pour ladite tête.

Une contre-vis est reçue et vissée dans ce lamage, en exerçant sur la tête de l'organe de fixation un effort axial de compression qui l'applique contre le fond du lamage.

Un tel principe de fixation, qui consiste à solidariser et bloquer la tête de vis relativement à l'implant par la contre-vis, permet de bénéficier d'un effet de *« scellement »* réalisé par l'ancrage de la vis dans l'os d'une part, et de la fixation de l'implant sur la vis d'autre part.

Ainsi, contrairement aux agencements plus classiques, la solidarisation de l'os et de l'implant décrit dans le document EP-345 133 ne dépend pas d'une force de compression qui les applique l'un contre l'autre, ni par conséquent des forces de frottement au niveau de l'interface implant / os.

Dès lors, même lorsque la matière osseuse se dégrade au niveau de l'interface implant / os, l'implant décrit dans le document EP-345 133 reste monté de façon stable, puisque le contact os / implant n'est pas impératif pour assurer la tenue du montage.

Ce caractère de stabilité favorise la reconstruction osseuse, et permet de minimiser les difficultés chirurgicales et post-opératoires, ce qui contribue à une remise en charge du patient dans les meilleures conditions.

Le dispositif décrit dans le document EP-345 133 présente cependant l'inconvénient de ne pouvoir fixer librement l'orientation de la vis relativement à l'implant.

Le dispositif décrit dans ce document n'autorise en effet qu'un positionnement angulaire unique de la vis relativement à l'implant.

Or, il est particulièrement intéressant de disposer d'un système orientable à volonté, en particulier en traumatologie, par exemple pour rapprocher un fragment osseux isolé.

De plus, dans le cas particulier où l'implant est une plaque d'ostéosynthèse, il s'avère généralement indiqué de varier les angles de vis de fixation relativement à la plaque, de façon à bénéficier d'ancrage selon des axes qui se croisent entre eux, ce qui limite les effets d'arrachement de la plaque lorsque cette dernière est soumise à des efforts mécaniques du genre traction normale, flexion ou autre.

Dans ce cas, il s'avère intéressant de pouvoir choisir, au moment de la pose, les angulations des vis, de façon à les adapter aux contraintes anatomiques particulières du patient opéré.

On connaît, par le document FR-2 790 198, un système d'implant permettant le détermination libre de l'angulation de la vis par rapport à l'implant.

Le système décrit dans ce document comprend un implant doté d'un orifice au sein duquel est positionnée une bague expansible radialement dans laquelle est vissée la vis de fixation.

Ladite vis présente un élargissement radial à proximité de sa tête, de sorte qu'en fin de vissage, cet élargissement provoque l'expansion radiale de la bague et le blocage de cette dernière en position contre les parois de l'orifice.

Un tel système ne permet cependant pas de bénéficier d'un effet de *« scellement »* équivalent à celui procuré par l'agencement décrit dans le document EP-345 133 précédemment cité.

De surcroît, le système décrit dans le document FR-2 790 198 nécessite l'utilisation à la fois d'un instrument de serrage de la vis et d'un instrument de blocage de la bague pendant le serrage de la vis. Par conséquent, les deux mains du chirurgien sont prises simultanément, ce qui complique la tâche opératoire.

En outre, l'agencement proposé par le document FR-2 790 198 expose à des expulsions intempestives de la bague hors de l'orifice, puisque la bague est retenue au sein de l'orifice uniquement et exclusivement par les forces de frottement générées par la pression radiale exercée par l'élargissement radial de la vis.

Enfin, dans le système décrit par le document FR-2 790 198, le blocage en position de la vis relativement à l'implant se fait dans le même temps que le vissage de la vis dans l'os, selon un geste de vissage unique. Outre le fait qu'une telle confusion des tâches ne permet pas de réaliser avec précision, en terme notamment d'intensité d'effort, le vissage dans l'os ainsi que le blocage en position angulaire, certains montages s'avèrent en pratique extrêmement délicats à réaliser. C'est le cas par exemple des montages *« en pilotis* », où l'implant, à l'issue du montage, est situé à une distance notable de l'os.

L'objet de l'invention vise en conséquence à porter remède aux différents inconvénients énumérés précédemment et à proposer un nouveau dispositif, notamment un implant chirurgical, muni d'un organe de fixation et destiné à être accouplé à au moins un support, notamment osseux, dont la conception permet de réaliser un assemblage particulièrement stable, tout en autorisant une orientation angulaire quelconque de l'organe de fixation relativement au support.

Un autre objet de l'invention vise à proposer un nouveau dispositif, notamment un implant chirurgical, muni d'un organe de fixation et destiné à être accouplé à au moins un support, notamment osseux, dont la conception facilite la recherche d'une orientation géométrique précise de l'organe de fixation.

Un autre objet de l'invention vise à proposer un nouveau dispositif, notamment un implant chirurgical, muni d'un organe de fixation et destiné à être accouplé à au moins un support, notamment osseux, dont la conception autorise un blocage de la tête de l'organe de fixation relativement au dispositif.

Un autre objet de l'invention vise à proposer un nouveau dispositif, notamment un implant chirurgical, muni d'un organe de fixation et destiné à être accouplé à au moins un support, notamment osseux, dont la conception permet une dissociation entre l'action d'ancrage de l'organe de fixation dans le support et l'action de blocage de l'organe de fixation dans une position angulaire déterminée.

Un autre objet de l'invention vise à proposer un nouveau dispositif, notamment un implant chirurgical, muni d'un organe de fixation et destiné à être accouplé à au moins un support, notamment osseux, qui présente un caractère démontable.

Un autre objet de l'invention vise à proposer un nouveau dispositif, notamment un implant chirurgical, muni d'un organe de fixation et destiné à être accouplé à au moins un support, notamment osseux, dont la mise en oeuvre est particulièrement sûre.

Un autre objet de l'invention vise à proposer un nouveau dispositif, notamment un implant chirurgical, muni d'un organe de fixation et destiné à être accouplé à au moins un support, notamment osseux, dont la fabrication est particulièrement simple et rapide et permet d'éviter tout désassemblage intempestif des éléments constitutifs du dispositif.

Les objets assignés à l'invention sont atteints à l'aide d'un dispositif destiné à être accouplé à au moins un support, ledit dispositif comprenant :
- un organe de fixation comportant une tête et une tige destinée à être ancrée dans le support,
- une pièce d'assemblage comprenant au moins un trou débouchant,
- une bague logée de manière orientable au sein dudit trou débouchant, ladite bague étant munie d'un passage conformé pour d'une part permettre le passage de la tige, et d'autre part pour réaliser un moyen d'arrêt pour la tête,
caractérisé en ce que la bague est expansible selon sensiblement sa direction axiale entre une configuration initiale dans laquelle la bague est orientable relativement au trou et une configuration expansée dans laquelle la bague est bloquée relativement au trou.

D'autres avantages et objets de l'invention apparaîtront plus en détails à la lecture de la description qui suit, et à l'aide des dessins annexés fournis à titre purement illustratif et non limitatif, dans lesquels :
- La figure 1 illustre, selon une vue écorchée en perspective, un exemple de réalisation d'un dispositif conforme à l'invention.
- La figure 2 illustre, selon une vue de côté en coupe, le dispositif représenté à la figure 1.
- La figure 3 illustre, selon une vue de dessus, une pièce d'assemblage faisant partie du dispositif conforme à l'invention représenté aux figures 1 et 2.
- La figure 4 illustre, selon une vue de côté, une bague faisant partie du dispositif conforme à l'invention représenté aux figures 1 et 2.
- La figure 5 illustre, selon une vue de dessus, une étape de fabrication du dispositif conforme à l'invention, dans laquelle la bague représentée à la figure 4 est insérée dans la pièce d'assemblage représentée à la figure 3.

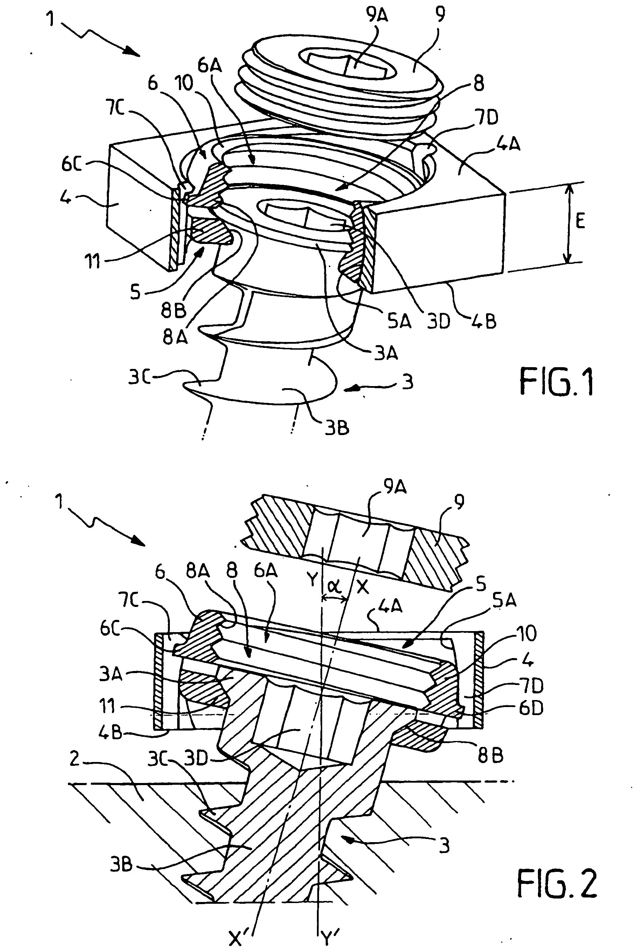

Les figures 1 et 2 montrent un dispositif 1 destiné à être accouplé à au moins un support 2, en vue de former un assemblage avec ledit support 2.

Dans ce qui suit, on fera référence à un dispositif 1 de nature chirurgicale, le support 2 étant constitué de matière osseuse.

Néanmoins, au sens de l'invention, le dispositif 1 conforme à l'invention pourra être de toute nature, de même que le support 2 auquel il est destiné à être fixé.

Le dispositif 1 conforme à l'invention comprend un organe de fixation 3 comportant une tête 3A ainsi qu'une tige 3B destinée à être ancrée dans le support 2.

Tel que cela est représenté aux figures 1 et 2, l'organe de fixation 3 est de préférence une vis. Dans ce cas, la tige 3B est munie de façon préférentielle d'un filetage 3C. La tête 3A comprend quant à elle, de façon préférentielle, une collerette tronconique (mieux visible sur la figure 2) se poursuivant par une zone lisse de forme générale sensiblement cylindrique. La tête 3A est préférentiellement munie d'une empreinte 3D, du genre empreinte à six pans, destinée à recevoir une clé de serrage correspondante en vue d'effectuer le vissage de la tige 3B dans le support 2.

Il est toutefois envisageable, sans pour autant sortir du cadre de l'invention, que l'organe de fixation 3 soit constitué par tout autre moyen alternatif, bien connu de l'homme du métier, et par exemple par un clou.

Le dispositif 1 conforme à l'invention comprend également une pièce d'assemblage 4 destinée à être solidarisée au support 2.

Dans une application préférentielle de l'invention, la pièce d'assemblage 4 est un implant ou ancillaire chirurgical, destiné à être associé à un support 2 de nature osseuse.

De façon préférentielle, la pièce d'assemblage 4 est une plaque chirurgicale d'ostéosynthèse destinée par exemple à la réduction d'une fracture osseuse.

Dans ce cas, la plaque 4 fait office de liaison entre les différents fragments de l'os fracturé, chaque fragment étant lié à la plaque 4 par l'intermédiaire d'au moins un organe de fixation 3.

Il est également envisageable, à titre d'exemple, que la pièce d'assemblage 4 soit constituée par un implant cotyloïdien.

Dans ce qui suit, on fera plus particulièrement référence à une pièce d'assemblage 5 constituée par un implant chirurgical, étant entendu que l'invention trouve son application dans de nombreux autres secteurs, tel que le bâtiment ou le bricolage par exemple.

Conformément à l'invention, la pièce d'assemblage 4 comprend au moins un trou débouchant 5. Ledit trou débouchant 5 est un orifice pratiqué de part en part de la pièce d'assemblage 4, dans toute son épaisseur E, entre sa face supérieure 4A et sa face inférieure 4B opposée. Ladite face inférieure 4B est destinée à être positionnée en regard du support 2.

Le dispositif 1 comprend également une bague 6, c'est-à-dire un élément annulaire, logée de manière orientable au sein du trou débouchant 5.

Le trou débouchant 5 constitue ainsi une cavité qui forme un logement accueillant la bague 6, de préférence de façon captive.

Par « *logée de manière orientable* », on désigne ici le fait que la direction axiale X-X' attachée à la bague 6 peut être déterminée de façon libre, par exemple par un chirurgien, et être notamment différente de la direction axiale Y-Y' attachée à la pièce d'assemblage 4. Les directions axiales X-X' et Y-Y' correspondent respectivement sensiblement à l'axe de symétrie de la bague 6 et du trou débouchant 5. Ainsi, dans la limite d'une plage prédéterminée, l'angle α présent entre les directions axiales respectives X-X', Y-Y' de la bague 6 et de la pièce d'assemblage 4 pourra être choisi et fixé à volonté par l'utilisateur.

La bague 6 est munie par ailleurs d'un passage 6A qui la traverse de part en part dans son épaisseur e. Ledit passage 6A est conformé pour d'une part permettre le passage de la tige 38 de l'organe de fixation 3, et d'autre part pour réaliser un moyen d'arrêt pour la tête 3A de l'organe de fixation 3.

Grâce à cet agencement, il est possible d'enfiler complètement l'organe de fixation 3 dans la bague 6 jusqu'à ce que la tête 3A soit stoppée par une conformation du passage 6A formant moyen d'arrêt. On comprend donc que de façon préférentielle, le diamètre hors-tout de la tige 3B de l'organe de fixation 3 est inférieur au diamètre minimum du passage 6A.

Conformément à une caractéristique importante de l'invention, la bague 6 est expansible selon sensiblement sa direction axiale X-X' entre d'une part une configuration initiale, dans laquelle la bague 6 conserve sa capacité à être orientée de façon libre relativement au trou, et d'autre part une configuration expansée, dans laquelle la bague 6 est bloquée dans une position angulaire déterminée relativement au trou débouchant 5. En d'autres termes, la bague 6 présente une capacité à se déformer axialement, c'est-à-dire à voir son épaisseur e augmenter pour l'amener d'une configuration initiale dans laquelle ladite bague 6 est orientable relativement au trou 5 à une configuration expansée dans laquelle la bague 6 est coincée, c'est-à-dire encastrée mécaniquement dans le trou débouchant 5.

Ainsi, le principe général de l'invention repose pour une bonne part sur la mise en oeuvre d'une bague 6 qui présente une capacité de changement géométrique de forme selon la direction axiale X-X' qui lui est attachée, cette expansion axiale venant modifier l'encombrement global de la bague 6, de telle sorte que cette dernière vient en butée contre la paroi 5A de la cavité formée par le trou débouchant 5, ce qui a pour effet de supprimer sensiblement tout degré de liberté entre la bague 6 et la pièce d'assemblage 4.

Le principe de l'invention repose donc sur une variation dimensionnelle dans la direction axiale X-X' de la bague 6 et non, comme dans l'art antérieur, sur la pression radiale exercée par un plot contre la paroi d'un trou débouchant.

Avantageusement, le trou débouchant 5 et la bague 6 sont conformés pour réaliser ensemble une liaison cinématique sphérique (appelée également rotule) entre la pièce d'assemblage 4 et la bague 6 lorsque cette dernière est en configuration initiale, c'est-à-dire non expansée.

De façon préférentielle, cette liaison sphérique peut être obtenue de façon classique en conformant le trou débouchant 5 de telle sorte que sa paroi interne 5A présente une forme sensiblement assimilable à une bande sphérique (c'est-à-dire une surface sphérique amputée de ses deux calottes polaires), la bague 6 présentant quant à elle un contour latéral extérieur 6B complémentaire ajustée à celui de la paroi interne 5A du trou débouchant 5.

Ainsi, lorsque les directions axiales X-X' et Y-Y' attachées respectivement à la bague 6 et au trou débouchant 5 sont alignées, le contour 6B de la bague 6 épouse sensiblement la paroi interne 5A.

De façon encore plus préférentielle, le trou débouchant 5 et la bague 6 sont conformés pour réaliser une liaison cinématique sphérique à doigt entre la bague 6 et la pièce d'assemblage 4 lorsque ladite bague 6 est en configuration initiale, ladite liaison sphérique à doigt interdisant sensiblement la rotation de la bague 6 autour de sa direction axiale X-X'.

En d'autres termes, la liaison sphérique à doigt permet les mêmes déplacements relatifs de la bague 6 et du trou 5 que la liaison sphérique évoquée précédemment, à l'exception du fait que cette fois, la rotation de la bague 6 sur elle-même autour de son axe de symétrie X-X' est sensiblement empêchée.

De façon préférentielle, cette liaison sphérique à doigt est réalisée de la même façon que la liaison sphérique précédemment décrite, à la différence près que la bague 6 comprend au moins un ergot radial 6C, et de préférence deux ergots radiaux 6C, 6D diamétralement opposés, tel que cela est illustré à la figure 6. Lesdits ergots radiaux 6C, 6D forment ainsi une excroissance relativement au contour externe latéral 6B de la bague 6. Chaque ergot radial 6C, 6D est destiné à coopérer avec une rainure correspondante 7C, 7D de forme complémentaire ménagée longitudinalement de façon sensiblement rectiligne, sur tout ou partie de la paroi latérale 5A du trou débouchant 5, de préférence parallèlement à la direction axiale Y-Y' attachée au trou 5 et à la pièce d'assemblage 4.

La coopération des ergots 6C, 6D avec les rainures correspondantes 7C, 7D selon sensiblement une liaison cinématique de type glissière permet ainsi d'interdire uniquement la rotation de la bague 6 autour de la direction axiale X-X' qui lui est attachée.

Il est cependant tout à fait envisageable, sans pour autant sortir du cadre de l'invention, de prévoir tout autre type de liaison cinématique entre la bague 6 et la pièce d'assemblage 4 permettant une angulation de ladite bague 6 relativement à la pièce 4. On pourra par exemple envisager une liaison cinématique libre entre la bague 6 et le trou débouchant 5, c'est-à-dire que la bague 6 est simplement contenue dans le trou débouchant 5, ce dernier n'assurant aucune fonction de guidage particulière. A titre d'exemple, on peut ainsi envisager que le trou débouchant 5 présente une section sensiblement rectangulaire, tandis que la bague présente un contour externe 6B partiellement ou totalement polyédrique, qui n'est donc pas complémentaire de celui de la paroi interne 5A du trou 5.

Avantageusement, le passage 6A ménagé dans la bague 6 comprend un lamage taraudé 8 définissant, de façon classique, d'une part un alésage taraudé 8A et d'autre part un fond 8B, lequel fond 8B forme surface d'appui et moyen d'arrêt pour la tête 3A de l'organe de fixation 3.

Ainsi, la tête 3A vient reposer en butée sur l'épaulement formant le fond 8B. Le diamètre de l'alésage taraudé 8A est ainsi préférentiellement supérieur au diamètre hors-tout de l'ensemble de l'organe de fixation 3, tandis que le diamètre du passage 6A au niveau de la restriction de section formant fond 8B est supérieur à celui de la tige 3B, mais inférieur à celui de la tête 3A.

Le dispositif 1 conforme à l'invention comprend également une contre-vis 9 destinée à être reçue et vissée dans le lamage 8 contre la tête 3A de l'organe de fixation 3, de telle sorte que ladite tête 3A est interposée entre le fond 8B et la contre-vis 9.

La contre-vis 9 se présente de préférence sous la forme d'un plot cylindrique fileté muni d'une empreinte de serrage 9A, laquelle est destinée à recevoir un outil de serrage.

De manière préférentielle, l'empreinte de serrage 3D de l'organe de fixation 3 et l'empreinte de serrage 9A de la contre-vis 9 sont de natures identiques, ce qui permet d'utiliser un seul et même outil pour effectuer d'une part l'ancrage de l'organe de fixation 3 dans le support 2 et d'autre part le serrage de la contre-vis 9.

La contre-vis 9 permet ainsi d'encastrer la tête 3A dans la bague 6.

De surcroît, le dispositif 1 est agencé de telle sorte que le vissage de la contre-vis 9 dans le lamage 8 engendre l'expansion axiale de la bague 6, de façon à amener cette dernière en configuration expansée.

Ainsi, dans ce mode de réalisation préférentiel de l'invention, l'expansion axiale de la bague 6 est obtenue par vissage d'une contre-vis 9.

Il est cependant tout à fait envisageable, sans pour autant sortir du cadre de l'invention, de mettre en oeuvre, d'autres moyens qu'une contre-vis 9 pour réaliser l'expansion axiale de la bague 6. On peut ainsi envisager que la bague 6 comporte une portion expansible, qui peut par exemple être gonflée par un fluide du genre liquide ou gaz, le gonflement de la portion expansible de la bague engendrant l'expansion axiale de ladite bague. On peut également envisager, dans un autre exemple de réalisation, que la bague 6 comporte une structure élastique, du genre ressort, précontrainte, de telle sorte que lorsque la précontrainte cesse, le ressort se détend ce qui conduit à l'expansion axiale de la bague 6. La bague 6 peut ainsi notamment être constituée de deux pièces rigides liées entre elles par un ressort de compression, les deux pièces étant plaquées l'une contre l'autre de façon à précontraindre le ressort, et étant interverrouillées dans cette position plaquée. Lorsque l'interverrouillage est désactivé, le ressort se détend, ce qui conduit à l'écartement des pièces et donc à l'expansion globale, dans la direction axiale, de la bague 6. L'homme du métier pourra mettre en oeuvre tout autre moyen bien connu pour réaliser une expansion axiale.

Avantageusement, la bague 6 comprend deux éléments, à savoir un premier et un deuxième éléments 10, 11, l'expansion sensiblement axiale de la bague vers sa configuration expansée correspondant à un écartement relatif desdits éléments 10, 11 selon sensiblement la direction axiale X-X'.

Lesdits éléments 10, 11 sont de préférence de forme annulaire, et sont disposés coaxialement l'un par rapport à l'autre pour former la bague 6.

L'expansion axiale correspond ainsi à un éloignement relatif du premier et du deuxième éléments 10, 11 selon un mouvement correspondant sensiblement à une translation dans la direction axiale X-X'. Cet écartement relatif modifie la forme du contour latéral général de la bague 6, qui passe par exemple d'une forme de bande sphérique autorisant la rotation de la bague 6 dans le trou débouchant 5, à une forme de bande non sphérique dont le profil se compose d'une première portion courbe 10A attachée au premier élément, d'une deuxième portion courbe 11A attachée au deuxième élément lesdites portions courbes 10A, 11A étant séparées par un espace majoritairement vide.

Cette forme de bande non sphérique, du fait qu'elle n'est plus complémentaire de la forme de la paroi interne 5A du trou 5, empêche tout mouvement de la bague 6 dans le trou 5.

Avantageusement, lesdits deux élément 10, 11 sont distincts et liés entre eux par au moins une patte de liaison 12 présentant un caractère souple de déformation.

Ainsi, le premier et le deuxième élément 10, 11 présentent de préférence un caractère sensiblement rigide, la capacité de déformation axiale de la bague 6 provenant en grande partie, voire en totalité, de la nature déformable dans la direction X-X' d'un plot formant patte de liaison 12.

Pour des raisons de symétrie, on privilégiera, dans ce mode de réalisation, la mise en oeuvre de deux pattes de fixation 12 diamétralement opposées, lesdites pattes de fixation 12 reliant lesdits éléments 10, 11 par leur périphérie.

A titre d'exemple, lesdites pattes de fixation 12 peuvent par exemple être constituées de plots élastiques (par exemple en matière plastique) ou encore de lames métalliques pliées en V, de telle sorte que le caractère souple des pattes de liaison 12 correspond à un dépliage desdites pattes 12.

Dans un mode alternatif de réalisation de l'invention, il est cependant tout à fait envisageable que le premier élément 10 et le deuxième élément 11 soient totalement indépendants l'un de l'autre, c'est-à-dire qu'ils ne soient pas liés mécaniquement par un élément de jonction du genre patte de liaison 12.

Avantageusement, le lamage taraudé 8 est réparti sur les deux éléments 10, 11 de façon à ce que l'alésage taraudé 8A appartienne, de préférence exclusivement, au premier élément 10, tandis que le fond 8B appartient, de préférence exclusivement, au deuxième élément 11.

De cette façon, lors du vissage de la contre-vis 9, cette dernière vient s'appliquer contre la tête 3A de l'organe de fixation 3 laquelle repose en appui contre le fond 8B. L'effort de vissage de la contre-vis 9 va ainsi produire une force de compression contre la tête 3A, ladite force engendrant, du fait de l'indépendance des éléments 10, 11 portant respectivement l'alésage taraudé 8A et le fond 8B, un écartement relatif de cesdits éléments 10, 11.

Avantageusement, lorsque l'organe de fixation 3 est une vis, le lamage 8 est taraudé selon un sens inverse à celui du filetage 3C de ladite vis.

Avantageusement, le premier élément 10 et le trou débouchant 5 sont conformés pour réaliser une liaison cinématique sphérique à doigt entre ledit premier élément 10 et la pièce d'assemblage 4 lorsque la bague 6 est en configuration initiale. Ladite liaison sphérique à doigt interdit sensiblement la rotation du premier élément 10 autour de la direction axiale X-X' qui lui est attachée.

La réalisation de cette liaison permet ainsi d'empêcher la rotation sur lui-même du premier élément 10 lorsque la contre-vis 9 est vissée dans le lamage 8 dudit premier élément 10.

Avantageusement, la liaison sphérique à doigt susdite est réalisée en pourvoyant le premier élément 10 d'au moins un ergot radial 6C, 6D destiné à coopérer avec une rainure correspondante 7C, 7D de forme complémentaire ménagée longitudinalement sur la paroi latérale 5A du trou débouchant 5, de façon à interdire sensiblement la rotation du premier élément 10 autour de sa direction axiale X-X'.

On obtient ainsi une reprise du couple de serrage de la contre-vis 9 par le dispositif 1 lui-même, sans nécessiter l'intervention de l'utilisateur, ni l'utilisation d'un outil spécifique. Les ergots radiaux 6C, 6D sont de préférence au nombre de deux, placés sur la surface latérale du premier élément 10 et faisant saillie radialement relativement à l'axe X-X' dudit premier élément 10.

Avantageusement, la section transversale 13 du trou débouchant 5 au niveau de la face supérieure 4A et/ou inférieure 4B est conformée pour permettre l'introduction dans le trou débouchant 5 de la bague 6 en configuration initiale, et ce uniquement lorsque ladite bague 6 est introduite par sa tranche 6B, à la manière d'une pièce de monnaie dans une tirelire.

A cette fin, la section transversale 13 dudit trou débouchant 5 au niveau de la face supérieure 4A et/ou inférieure 4B, présente de façon préférentielle une forme générale sensiblement circulaire, avec deux dégagements supplémentaires 14, 15 diamétralement opposés.

Lesdits dégagements supplémentaires 14, 15 peuvent être complétés par les rainures 7C, 7D, lorsque la bague 6 est munie d'ergots radiaux 6C, 6D.

Ainsi, du fait que le diamètre D de la portion circulaire de la section transversale 13 est inférieur au diamètre hors-tout d de la bague 6, cette dernière ne peut être insérée complètement au sein du trou débouchant 5 que si elle est introduite verticalement, c'est-à-dire par son flanc latéral 6B, dans la zone s'étendant entre les dégagements 14, 15.

De cette façon, une fois la bague 6 complètement insérée dans le trou débouchant 5, ladite bague 6 ne pourra être en mesure de s'échapper hors dudit trou 5 que si elle est placée dans une positon identique à sa position d'introduction.

Or, une telle position d'introduction ne pourra plus être atteinte une fois que l'organe de fixation 3 est enfilé dans la bague 6.

L'agencement décrit précédemment permet ainsi de maintenir de façon passive la bague 6 captive dans le trou débouchant 5, que cette bague soit constituée d'un ou plusieurs éléments, indépendants ou non.

La configuration décrite précédemment, qui correspond d'ailleurs à celle illustrée aux figures, n'est cependant donnée qu'à titre d'exemple.

En lieu et place d'une section transversale 13 de forme générale circulaire, il est ainsi tout à fait envisageable de prévoir une section transversale 13 de forme rectangulaire ou carrée, ou encore polygonale.

Le principe général de l'invention repose en effet sur le fait que les sections extrêmes 13 correspondants aux faces supérieure 4A et inférieure 4B de la pièce d'assemblage 4 présentent une forme interdisant le passage de la bague 6 autrement que selon une orientation prédéterminée, en l'occurrence sur la tranche 6B.

La réalisation et le fonctionnement de l'invention vont maintenant être décrits en se basant sur l'exemple d'un dispositif chirurgical.

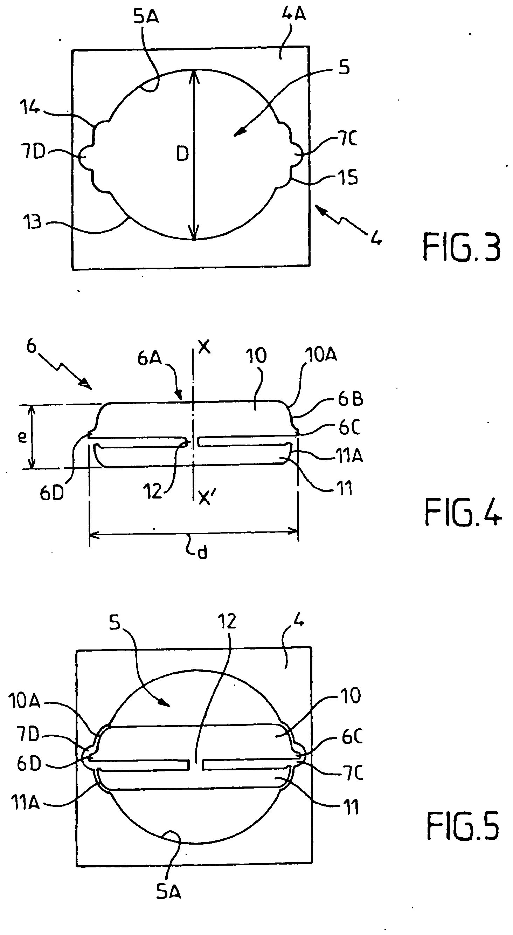

Une bague 6 conforme à l'invention est introduite par sa tranche 6B dans le trou débouchant 5 d'une plaque d'ostéosynthèse 4, tel que cela est illustré à la figure 5.

Une fois la bague 6 introduite complètement dans le trou débouchant 5, ladite bague 6 est ensuite pivotée autour de l'axe passant par ses ergots axiaux 6C, 6D pour se retrouver en position de réception d'une vis d'ostéosynthèse 3. Ainsi, on réalise une liaison sphérique à doigt par l'introduction dans un logement 5 d'une pièce unique, en l'occurrence la bague 6, laquelle est introduite sans déformation dans le trou débouchant 5, ce qui limite le risque d'expulsion de la bague 6 en service. A ce stade, le lamage taraudé 8 de la bague 6 et le trou débouchant 5 sont sensiblement coaxiaux, ou du moins proches d'une telle coaxialité. La vis d'ostéosynthèse 3 est alors enfilée à la fois dans le trou débouchant 5 et dans le lamage taraudé 8 jusqu'à venir en butée par sa tête 3A contre le fond 8B dudit lamage taraudé 8.

Selon l'exemple présenté aux figures, l'axe de la vis 3 et l'axe X-X' de la bague 6 sont alors sensiblement confondus. Il est cependant envisageable, sans pour autant sortir du cadre de l'invention, que tel ne soit pas le cas, ce qui pourrait se produire si l'on envisage que l'axe du lamage fileté 8 diffère de celui de la bague 6.

Le praticien introduit ensuite un outil de serrage dans l'empreinte 3D de la vis et peut visser cette dernière dans le support osseux 2 en choisissant l'angle entre ladite vis 3 et la plaque 4, puisque la vis 3 et la bague 6 forment un sous-ensemble mobile angulairement relativement à la plaque 4.

Une fois l'ancrage de la vis 3 effectuée au sein de l'os 2, le chirurgien procède alors au verrouillage en position angulaire de la plaque 4 relativement à la vis 3, ainsi qu'au scellement de la tête de vis 3A, par le vissage dans le lamage taraudé 8 d'une contre-vis 9. La contre-vis 9 va venir prendre appui sur le sommet de la tête 3A puis progressivement écarter le premier élément 10 du deuxième élément 11 formant la bague 6. La contre-vis 9 présente ainsi une double fonction, à savoir d'une part une fonction de blocage de la tête de vis 3A, puisque cette dernière sera prise en compression entre le fond 8B et la contre-vis 9, et d'autre part une fonction d'expansion axiale de la bague 6, puisque une fois la contre-vis 9 arrivée en butée contre la tête de vis 3A, tout vissage supplémentaire provoque par réaction l'écartement des éléments 10, 11 constituant la bague 6. Le serrage de la contre-vis 9 s'effectue à l'aide d'un outil tout à fait standard, et ne nécessite pas de maintien de la bague 6, puisque cette dernière est bloquée en rotation axiale par les ergots 6C, 6D. Les gestes de serrage de la vis 3 et de la contre-vis 9 sont donc dissociés.

Le dispositif conforme à l'invention permet ainsi de bénéficier d'un montage particulièrement stable et résistant notamment aux forces d'arrachement, de cisaillement ou d'enfoncement.

Le dispositif conforme à l'invention s'avère notamment particulièrement efficace dans le cas où le matériau du support 2 présente une dureté moindre que celle de la pièce d'assemblage 4.

Enfin, l'agencement proposé est entièrement démontable.

## Revendications

1. Dispositif chirurgical (1), du genre implant, destiné à être accouplé à au moins un support (2), ledit dispositif (1) comprenant :
- un organe de fixation (3) comportant une tête (3A) et une tige (3B) destinée à être ancrée dans le support (2),
- une pièce d'assemblage (4) comprenant au moins un trou débouchant (5),
- une bague (6) logée de manière orientable au sein dudit trou débouchant (5), ladite bague (6) étant munie d'un passage (6A) conformé pour d'une part permettre le passage de la tige (3B) et d'autre part pour réaliser un moyen d'arrêt pour la tête (3A),
**caractérisé en ce que** la bague (6) est expansible selon sensiblement sa direction axiale (X-X') entre une configuration initiale dans laquelle la bague (6) est orientable relativement au trou (5) et une configuration expansée dans laquelle la bague (6) est bloquée relativement au trou (5).

2. Dispositif (1) selon la revendication 1 **caractérisé en ce que** le trou débouchant (5) et la bague (6) sont conformés pour réaliser une liaison cinématique sphérique entre la pièce d'assemblage (4) et la bague (6) lorsque la bague (6) est en configuration initiale.

3. Dispositif (1) selon la revendication 1 **caractérisé en ce que** le trou débouchant (5) et la bague (6) sont conformés pour réaliser une liaison cinématique sphérique à doigt entre la bague (6) et la pièce d'assemblage (4) lorsque la bague (6) est en configuration initiale, ladite liaison sphérique à doigt interdisant sensiblement la rotation de la bague (6) autour de sa direction axiale X-X'.

4. Dispositif (1) selon la revendication 3 **caractérisé en ce que** la bague (6) comprend au moins un ergot radial (6C, 6D) destiné à coopérer avec une rainure correspondante (7C, 7D) de forme complémentaire ménagée longitudinalement sur la paroi latérale (5A) du trou débouchant (5), de façon à interdire sensiblement la rotation de la bague (6) autour de sa direction axiale (X-X').

5. Dispositif (1) selon l'une des revendications 1 à 4 **caractérisé en ce que** le passage (6A) comprend un lamage taraudé (8) définissant un alésage taraudé (8A) et un fond (8B), lequel fond (8B) forme surface d'appui et moyen d'arrêt pour ladite tête (3A), ledit dispositif comprenant une contre-vis (9) destinée à être vissée dans le lamage (8) contre ladite tête (3A), ce vissage engendrant l'expansion axiale de la bague (6).

6. Dispositif (1) selon l'une des revendications 1 à 5 **caractérisé en ce que** la bague (6) comprend deux éléments, à savoir un premier et un deuxième éléments (10, 11) superposés, l'expansion sensiblement axiale de la bague (6) vers sa configuration expansée correspondant à un écartement relatif desdits éléments (10, 11) selon sensiblement la direction axiale (X-X').

7. Dispositif selon la revendication 6 **caractérisé en ce que** lesdits deux éléments (10, 11) sont distincts et liés entre eux par au moins une patte de liaison (12) présentant un caractère souple.

8. Dispositif (1) selon la revendication 6 **caractérisé en ce que** lesdits deux éléments (10, 11) sont indépendants l'un de l'autre.

9. Dispositif (1) selon la revendication 5 et l'une des revendications 6 à 8 **caractérisé en ce que** le lamage taraudé (8) est réparti sur les deux éléments (10, 11) de façon à ce que l'alésage taraudé (8A) appartienne au premier élément (10) tandis que le fond (8B) appartient au deuxième élément (11).

10. Dispositif (1) selon l'une des revendications 6 à 9 **caractérisé en ce que** ledit premier élément (10) et le trou débouchant (5) sont conformés pour réaliser une liaison cinématique sphérique à doigt entre le premier élément (10) et la pièce d'assemblage (4) lorsque la bague (6) est en configuration initiale, ladite liaison sphérique à doigt interdisant sensiblement la rotation du premier élément (10) autour de sa direction axiale (X-X').

11. Dispositif (1) selon la revendication 10 **caractérisé en ce que** le premier élément (10) comprend au moins un ergot radial (6C, 6D) destiné à coopérer avec une rainure correspondante (7C, 7D) de forme complémentaire ménagée longitudinalement sur la paroi latérale (5A) du trou débouchant (5), de façon à interdire sensiblement la rotation du premier élément (10) autour de sa direction axiale (X-X').

12. Dispositif (1) selon l'une des revendications 1 à 11 **caractérisé en ce que** le trou débouchant (5) est ménagé entre une face supérieure (4A) et une face inférieure (4B) opposée de la pièce d'assemblage (4), la section transversale (13) dudit trou (5) au niveau de la face supérieure (4A) et / ou inférieure (4B) étant conformée pour permettre l'introduction dans le trou débouchant (5) de la bague (6) en configuration initiale uniquement lorsque ladite bague (6) est introduite par sa tranche (6B).

13. Dispositif (1) selon la revendication 12 **caractérisé en ce que** la section transversale (13) dudit trou (5) au niveau de la face supérieure (4A) et / ou inférieure (4B) présente une forme générale sensiblement circulaire, avec deux dégagements supplémentaires (14, 15) diamétralement opposés.

14. Dispositif (1) selon l'une des revendications 1 à 13 **caractérisé en ce que** l'organe de fixation (3) est une vis.

15. Dispositif (1) selon les revendications 5 et 14 **caractérisé en ce que** le lamage (8) est taraudé selon un sens inverse à celui du filetage (3C) de la vis (3).

16. Dispositif (1) selon l'une des revendications 1 à 15 **caractérisé en ce que** ledit au moins un support (2) est de nature osseuse, tandis que la pièce d'assemblage (4) est un implant ou un ancillaire chirurgical.

17. Dispositif (1) selon la revendication 16 **caractérisé en ce que** la pièce d'assemblage (2) est une plaque d'ostéosynthèse.

## Patentansprüche

1. Chirurgische Vorrichtung (1), vom Typ eines Implantats, die dazu bestimmt ist, mit mindestens einem Halter (2) verbunden zu werden, wobei die Vorrichtung (1) Folgendes aufweist:
- ein Befestigungsorgan (3), das einen Kopf (3A) und eine Stange (3B) aufweist, die dazu bestimmt ist, in dem Halter (2) verankert zu werden,
- ein Verbindungsstück (4), das mindestens ein Durchgangsloch (5) aufweist,
- einen Ring (6), der drehbar in dem Durchgangsloch (5) gelagert ist, wobei der Ring (6) mit einem Durchgang (6A) ausgestattet ist, der ausgebildet ist, um einerseits das Durchgehen der Stange (3B) zu ermöglichen und andererseits, um ein Arretiermittel für den Kopf (3A) zu bilden,
**dadurch gekennzeichnet, dass** der Ring (6) im Wesentlichen entlang seiner axialen Richtung (X-X') zwischen einer Anfangskonfiguration, in der der Ring (6) relativ zu dem Loch (5) drehbar ist, und einer ausgedehnten Konfiguration, in der der Ring (6) relativ zu dem Loch (5) blockiert ist, ausdehnbar ist.

2. Vorrichtung (1) nach Anspruch 1, **dadurch gekennzeichnet, dass** das Durchgangsloch (5) und der Ring (6) ausgebildet sind, um eine sphärische, kinematische Verbindung zwischen dem Verbindungsstück (4) und dem Ring (6) zu bilden, wenn sich der Ring (6) in der Anfangskonfiguration befindet.

3. Vorrichtung (1) nach Anspruch 1, **dadurch gekennzeichnet, dass** das Durchgangsloch (5) und der Ring (6) ausgebildet sind, um eine sphärische, kinematische Verbindung mit Finger zwischen dem Ring (6) und dem Verbindungsstück (4) zu bilden, wenn sich der Ring (6) in der Anfangskonfiguration befindet, wobei die sphärische Verbindung mit Finger im Wesentlichen das Drehen des Rings (6) um seine axiale Richtung X-X' unmöglich macht.

4. Vorrichtung (1) nach Anspruch 3, **dadurch gekennzeichnet, dass** der Ring (6) mindestens einen radialen Stift (6C, 6D) aufweist, der dazu bestimmt ist, mit einer entsprechenden Nut (7C, 7D) von komplementärer Form zusammenzuwirken, die in Längsrichtung auf der Seitenwand (5A) des Durchgangslochs (5) derart angeordnet ist, um im Wesentlichen das Drehen des Rings (6) um seine axiale Richtung (X-X') unmöglich zu machen.

5. Vorrichtung (1) nach einem der Ansprüche 1 bis 4, **dadurch gekennzeichnet, dass** der Durchgang (6A) eine Senkung mit Gewinde (8) aufweist, die eine Gewindebohrung (8A) und einen Boden (8B) definiert, wobei der Boden (8B) Auflagefläche und Arretiermittel für den Kopf (3A) bildet, wobei die Vorrichtung eine Gegenschraube (9) aufweist, die dazu bestimmt ist, in die Senkung (8) gegen den Kopf (3A) geschraubt zu werden, wobei dieses Schrauben das axiale Ausdehnen des Rings (6) erzeugt.

6. Vorrichtung (1) nach einem der Ansprüche 1 bis 5, **dadurch gekennzeichnet, dass** der Ring (6) zwei Elemente aufweist, das heißt ein erstes und ein zweites Element (10, 11), die übereinanderliegend sind, wobei das im Wesentlichen axiale Ausdehnen des Rings (6) zu seiner ausgedehnten Konfiguration einem relativen Abstand der Elemente (10, 11) im Wesentlichen in der axialen Richtung (X-X') entspricht.

7. Vorrichtung nach Anspruch 6, **dadurch gekennzeichnet, dass** die zwei Elemente (10, 11) getrennt sind und durch mindestens eine Verbindungslasche (12) miteinander verbunden sind, die einen flexiblen Charakter aufweist.

8. Vorrichtung (1) nach Anspruch 6, **dadurch gekennzeichnet, dass** die zwei Elemente (10, 11) voneinander unabhängig sind.

9. Vorrichtung (1) nach Anspruch 5 und nach einem der Ansprüche 6 bis 8, **dadurch gekennzeichnet, dass** die Senkung mit Gewinde (8) auf den zwei Elementen (10, 11) derart verteilt ist, dass die Senkung mit Gewinde (8A) zu dem ersten Element (10) gehört, während der Boden (8B) zu dem zweiten Element (11) gehört.

10. Vorrichtung (1) nach einem der Ansprüche 6 bis 9, **dadurch gekennzeichnet, dass** das erste Element (10) und das Durchgangsloch (10) ausgebildet sind, um eine sphärische, kinematische Verbindung mit Finger zwischen dem ersten Element (10) und dem Verbindungsstück (4) zu bilden, wenn sich der Ring (6) in der Anfangskonfiguration befindet, wobei die sphärische Verbindung mit Finger im Wesentlichen das Drehen des ersten Elements (10) um seine axiale Richtung (X-X') unmöglich macht.

11. Vorrichtung (1) nach Anspruch 10, **dadurch gekennzeichnet, dass** das erste Element (10) mindestens einen radialen Stift (6C, 6D) aufweist, der dazu bestimmt ist, mit einer entsprechenden Nut (7C, 7D) von komplementärer Form zusammenzuwirken, die in Längsrichtung auf der Seitenwand (5A) des Durchgangslochs (5) derart angeordnet ist, um im Wesentlichen das Drehen des ersten Elements (10) um seine axiale Richtung (X-X') unmöglich zu machen.

12. Vorrichtung (1) nach einem der Ansprüche 1 bis 11, **dadurch gekennzeichnet, dass** das Durchgangsloch (5) zwischen einer Oberseite (4A) und einer Unterseite (4B), die dem Verbindungsstück (4) gegenüberliegt, angeordnet ist, wobei der Querschnitt (13) des Lochs (5) an der Oberseite (4A) und/oder Unterseite (4B) ausgebildet ist, um das Einführen des Rings (6) in der Anfangskonfiguration in das Durchgangsloch (5) nur dann zu ermöglichen, wenn der Ring (6) durch seinen Rand (6B) eingeführt wird.

13. Vorrichtung (1) nach Anspruch 12, **dadurch gekennzeichnet, dass** der Querschnitt (13) des Lochs (5) an der Oberseite (4A) und/oder Unterseite (4B) eine im Wesentlichen kreisförmige Form mit zwei zusätzlichen, diametral gegenüberliegenden Aussparungen (14, 15) aufweist.

14. Vorrichtung (1) nach einem der Ansprüche 1 bis 13, **dadurch gekennzeichnet, dass** das Befestigungsorgan (3) eine Schraube ist.

15. Vorrichtung (1) nach einem der Ansprüche 5 bis 14, **dadurch gekennzeichnet, dass** die Senkung (8) mit einem Gewinde in einer umgekehrten Richtung zu jener des Gewindes (3C) der Schraube (3) ist.

16. Vorrichtung (1) nach einem der Ansprüche 1 bis 15, dadurch gekenntzeichnet, dass der mindestens eine Halter (2) von knöcherner Beschaffenheit ist, während das Verbindungsstück (4) ein Implantat oder eine chirurgische Hilfsvorrichtung ist.

17. Vorrichtung (1) nach Anspruch 16, **dadurch gekennzeichnet, dass** das Verbindungsstück (4) eine Osteosyntheseplatte ist.

## Claims

1. Surgical device (1), of the implant type, destined to be coupled to at least one support (2), said device (1) including:
- a fixing element (3) including a head (3A) and a pin (3B) destined to be anchored in the support (2),
- an assembly part (4) including at least one through hole (5),
- a ring (6) housed in a manner that can be oriented within said through hole (5), said ring (6) being equipped with a passage (6A) shaped on the one hand to allow passage of pin (3B) and on the other hand to realise a means of stoppage for head (3A),
**characterised in that** ring (6) is expandable according to substantially its axial direction (X-X') between an initial configuration wherein ring (6) can be oriented relative to hole (5) and an expanded configuration wherein ring (6) is blocked relative to hole (5).

2. Device (1) according to claim 1 **characterised in that** through hole (5) and ring (6) are shaped to realise a spherical kinematic joint between assembly part (4) and ring (6) when ring (6) is in initial configuration.

3. Device (1) according to claim 1 **characterised in that** through hole (5) and ring (6) are shaped to realise a spherical kinematic joint catch between ring (6) and assembly part (4) when ring (6) is in initial configuration, said spherical joint catch substantially prohibiting the rotation of ring (6) around its axial direction X-X'.

4. Device (1) according to claim 3 **characterised in that** ring (6) includes at least one radial fin (6C, 6D) destined to cooperate with a corresponding groove (7C, 7D) of complementary shape arranged longitudinally on the lateral wall (5A) of through hole (5), such as to substantially prohibit the rotation of ring (6) around its axial direction (X-X').

5. Device (1) according to any of claims 1 to 4 **characterised in that** passage (6A) includes a countersink with internal threading (8) defining a bore with internal threading (8A) and a root (8B), which root (8B) forms a support surface and a means of stoppage for said head (3A), said device including a check screw (9) destined to be tightened into countersink (8) against said head (3A), this tightening causing axial expansion of ring (6).

6. Device (1) according to any of claims 1 to 5 **characterised in that** ring (6) includes two items, i.e. a first item and a second item (10, 11) superimposed, substantially axial expansion of ring (6) towards its expanded configuration corresponding to a relative separation of said items (10, 11) according to the axial direction substantially (X-X').

7. Device according to claim 6 **characterised in that** said two items (10, 11) are distinct and connected together by at least one bracket (12) that has a flexible nature.

8. Device (1) according to claim 6 **characterised in that** said two items (10, 11) are independent one from the other.

9. Device (1) according to claim 5 and any of claims 6 to 8 **characterised in that** countersink with internal threading (8) is distributed over the two items (10, 11) such that the bore with internal threading (8A) belongs to the first item (10) while root (8B) belongs to the second item (11).

10. Device (1) according to any of claims 6 to 9 **characterised in that** said first item (10) and through hole (5) are shaped to realise a spherical kinematic joint catch between the first item (10) and the assembly part (4) when ring (6) is in initial configuration, said spherical joint prohibiting substantially the rotation of the first item (10) around its axial direction (X-X').

11. Device (1) according to claim 10 **characterised in that** first item (10) includes at least one radial fin (6C, 6D) destined to cooperate with a corresponding groove (7C, 7D) of complementary shape arranged longitudinally on the lateral wall (5A) of through hole (5), such as to substantially prohibit the rotation of the first item (10) around its axial direction (X-X').

12. Device (1) according to any of claims 1 to 11 **characterised in that** through hole (5) is arranged between an upper side (4A) and an opposite lower side (4B) of the assembly part (4), transversal section (13) of said hole (5) at the level of the upper side (4A) and/or lower side (4B) being shaped to allow the introduction in through hole (5) of ring (6) in initial configuration only when said ring (6) is introduced via its edge (6B).

13. Device (1) according to claim 12 **characterised in that** the transversal section (13) of said hole (5) at the level of the upper side (4A) and/or lower side (4B) has a substantially circular general shape, with two additional undercuts (14, 15) diametrically opposed.

14. Device (1) according to any of claims 1 to 13 **characterised in that** the fixing element (3) is a screw.

15. Device (1) according to claims 5 and 14 **characterised in that** countersink (8) is threaded internally according to a direction that is inverse to that of the external threading (3C) of screw (3).

16. Device (1) according to any of claims 1 to 15 **characterised in that** at least one support (2) is of a bone nature, while assembly part (4) is an implant or surgical ancillary.

17. Device (1) according to claim 16 **characterised in that** assembly part (4) is an osteosynthesis plate.
